# EUROPEAN PATENT SPECIFICATION

(11) **EP 1 795 163 B1**
(45) Date of publication and mention of the grant of the patent: **14.11.2012**
(21) Application number: 05785211.3
(22) Date of filing: 21.09.2005
(51) Int. Cl.: A61F 13/49, A61F 13/56, A61F 13/62, A61F 13/15, A61F 13/496

(54) **ABSORPTIVE ARTICLE**
SAUGFÄHIGER ARTIKEL
ARTICLE ABSORBANT

(30) Priority: 22.09.2004 JP 2004275066; 18.10.2004 JP 2004302399
(43) Date of publication of application: 13.06.2007
(60) Divisional application: 12184207.4
(73) Proprietor: Kao Corporation, Chuo-ku, Tokyo 103-8210 (JP)
(72) Inventor: KOBAYASHI, Kenji, Haga-gun, Tochigi 3213497 (JP); ANDO, Kenji, Haga-gun, Tochigi 3213497 (JP); YANASHIMA, Takuo, Haga-gun, Tochigi 3213497 (JP); ENDO, Masanori, Haga-gun, Tochigi, 3213497 (JP)
(74) Representative: Vossius & Partner
(86) International application number: PCT/JP2005/017382
(87) International publication number: WO 2006/033350

(56) References cited:
- EP-A1- 1 669 048
- WO-A1-03/057116
- WO-A2-02/26182
- JP-A- 2000 502 573
- JP-A- 2002 532 196
- JP-A- 2003 144 493
- JP-A- 2005 095 588
- JP-U- 6 055 623

## Description

### Technical Field

The present invention relates to an absorbent article and a method of producing the absorbent article. More particularly, it relates to an absorbent article including a liquid permeable topsheet, a liquid impermeable leak preventive sheet, and a liquid retentive absorbent member between the sheets and having both side edge portions of the rear portion and both side edge portions of the front portion joined to each other to take on the form of a pair of pants, and a method making the same.

### Background of the Invention

Absorbent articles such as disposable diapers and pants-type diapers (pull-on diapers) are composed of, for example, a substantially oblong absorbent body and an exterior material including a rear portion, a front portion, and a crotch portion, the absorbent body having a liquid permeable topsheet, a liquid impermeable leak-preventive sheet, and a liquid retentive absorbent member. Various devices have been added so that bodily wastes may be effectively absorbed by the absorbent member and that the absorbed wastes may be retained without leakage.

A disposable diaper or a pull-on diaper is shaped into the form of an undergarment in conformity to the contour of the body of a wearer, such as a child or an elderly person, by folding the exterior material into two with the absorbent body inside and joining both side edges of the rear portion and those of the front portion to each other. To improve feel to the touch and body fit, waist elastic members, below-waist elastic members, leg elastic members, etc. are disposed on the exterior material.

A means generally practiced to connect the side edges of the rear portion to the side edges of the front portion includes overlapping the front and rear side edge portions and heat-sealing the overlap. Articles are also known, which are provided with a mechanical fastener composed of a male member and a female member as a refastenable joining means helping disjoin the side seam and fold the article compact for disposal or rejoin the disjoined side seam when necessary. When in using a mechanical fastener, if a facing pair of side edge portions 70a and 70b are joined together with a male member 71 and a female member 72 disposed on the inner side thereof as illustrated in Fig. 16, the joint strength will be too small to withstand a pulling force during wear that will be imposed in the direction of T. The way of joining the side edge portions shown in Fig. 17 is conceivable, in which the outer side of one of the side edge portions (side edge portion 73a) and the inner side of the other side edge portion (side edge portion 73b) are made to face each other and joined together via a male member 74 and a female member 75. In this case, the pulling force during wear is applied in the shear direction. However, the operation of making the inner side of the side edge portion 73b face the outer side of the other side edge portion 73a is a complex process in the continuous production of absorbent articles. In the case of a so-called transverse feed line in which an assembly web (continuous length) of an exterior material having a plurality of absorbent bodies spacedly disposed thereon is fed in the width direction of the absorbent articles to be manufactured, the operation of making the inner side of a side edge portion face the outer side of a facing side edge portion involves particular difficulty. This is because elastic members are arranged in a configuration ready to contract in the body circumferential direction and, after cutting the assembly web into individual articles, the above-described operation involves handling of contracted parts.

To address the above problems, an absorbent article and a process of making it have been developed, in which a male member and a female member are attached to the inner side of a facing pair of side edge portions, respectively, and yet a pulling force is imposed in the shear direction during wear (see, e.g., JP 2003-533278A). According to JP 2003-533278A, in manufacturing an absorbent article, a female member 76 of a mechanical fastener is folded along folding line 76c into two panels to have a flat C shape, disposed with the folding line 76c facing outward, and bonded on one of its panel (panel 76a) to the inner side of one of a pair of facing side edge portions (side edge portion 77a) with the folding line 76c outward, as shown in Fig. 18. The outer side of the other panel 76b is adapted to be engaged with a male member 78 that is attached to the inner side of the other side edge portion 77b.

In the manner of joining the side edge portion 77b of the rear portion and the side edge portion 77a of the front portion of an absorbent article according to JP 2003-533278A, the female member 76, being attached as folded in two, needs double the effective width, which is waste of material. Furthermore, the production of the absorbent article requires an operation of folding the female member 76 in two, which makes the production process complicated.

An object of the present invention is to provide an absorbent article whose rear side edge portion and front side edge portion are joined on their inner side via the respective male member and female member of a mechanical fastener such that a pulling force during wear may be imposed in the shearing direction and which absorbent article can be produced through simple steps without wasteful use of the female member; and a method of producing the absorbent article.

This object is achieved with the features of the claims.

### Disclosure of the Invention

The present invention provides an absorbent article having a liquid permeable topsheet, a liquid impermeable leak-preventive sheet, a liquid retentive absorbent member intermediate between the sheets, and an exterior material extending laterally outward of both rear and front portions of the absorbent member. The absorbent article has a side edge portion of the rear portion thereof and a side edge portion of the front portion thereof joined to each other via a mechanical fastener composed of a male member and a female member and therefore takes on the shape of a pair of pants. The male member is attached to the inner side (skin facing side) of the side edge portion of either the rear portion or the front portion. The female member is a sheet member attached, on the reverse side (the side facing the exterior material) of its outer edge portion thereof, to the inner side (skin facing side) of the side edge portion of the other. The portion of the female member inward of the outer edge portion is left free as a flap. The male member engages the flap of the female member to join the side edge portion of the rear portion and the side edge portion of the front portion with each other

The present invention also provides a method of producing the above-described absorbent article. The method includes the steps of continuously feeding an assembly web constituting the above-described absorbent article in a transverse feed line, folding the web into two along a folding line parallel with the feed direction, and cutting the folded web along predetermined cutting lines into individual absorbent articles. The method further includes, before the steps of folding and cutting, the step of attaching either the male or female member to both sides of each predetermined cutting line in either the rear or front portion of the assembly web and attaching the other member to both sides of each predetermined cutting line in the other portion of the assembly web. The sites where the male and female members are attached correspond to the inner side of both side edge portions of the rear and front portions of each absorbent article to be produced on cutting. In the subsequent step of folding the web into two, each male member engages the flap of each female member. On cutting the folded web, there are produced absorbent articles with the side edge portions of the rear portion and those of the front portion joined to each other via the respective mechanical fasteners.

### Brief Description of the Drawings

Fig. 1 is a front view of a pants-type diaper as an absorbent article incorporating a first embodiment of the present invention.
Fig. 2 is a front view of the pants-type diaper as an absorbent article incorporating the first embodiment of the invention, being in a state before both side edge portions of the diaper are joined together.
Fig. 3 is a schematic perspective view showing the essential part of the pants-type diaper as an absorbent article incorporating the first embodiment of the invention.
Fig. 4(a) is a fragmentary perspective view showing the essential part of the pants-type diaper as an absorbent article incorporating the first embodiment of the invention.
Fig. 4(b) is a cross-sectional view taken along line D-D of Fig. 4(a).
Fig. 5 schematically illustrates the steps involved in the production of the pants-type diaper as an absorbent article incorporating the first embodiment of the invention.
Fig. 6 is a plan view illustrating the essential part of the production of the pants-type diaper as an absorbent article incorporating the first embodiment of the invention.
Fig. 7 is a perspective view of the essential part of another pants-type diaper as an absorbent article incorporating the first embodiment of the invention.
Fig. 8 illustrates a conventional manner of joining with a mechanical fastener.

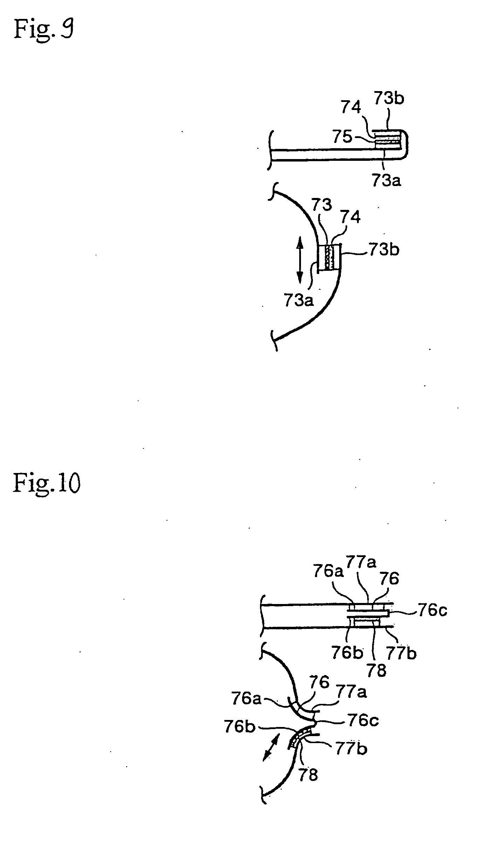

Fig. 9 illustrates another conventional manner of joining with a mechanical fastener.
Fig. 10 illustrates still another conventional manner of joining with a mechanical fastener.

### Detailed Description of the Invention

The present invention accomplishes the above object by the provision of an absorbent article having a liquid permeable topsheet, a liquid impermeable leak-preventive sheet, a liquid retentive absorbent member interposed between the sheets, and an exterior material extending laterally outward of both rear and front portions of the absorbent member. The absorbent article has a side edge portion of the rear portion and a side edge portion of the front portion joined via a mechanical fastener composed of a male member and a female member and therefore takes on a shape of a pair of pants. The male member is attached to the inner side (skin facing side) of the side edge portion of either the rear or front portion. The female member is a sheet member attached, on the reverse side (the side facing the exterior material) of its outer edge portion, to the inner side (skin facing side) of the side edge portion of the other of the rear and front portions. The portion of the female member inward of the outer edge portion is left free as a flap. The male member engages the flap of the female member to join the side edge portion of the rear portion and the side edge portion of the front portion with each other.

The present invention accomplishes the above object also by the provision of a method of producing the above-described absorbent article. The method includes the steps of continuously feeding an assembly web constituting the absorbent article in a transverse feed line, folding the web into two along a folding line parallel with the feed direction, and cutting the folded web into individual absorbent articles along predetermined cutting lines. The method further includes, before the steps of folding and cutting, the step of attaching the male member and the female member to the inner side of the assembly web on both sides of each predetermined cutting line corresponding to both side edge portions of either one of the rear and front portions and the other, respectively, of an absorbent article to be produced on cutting. In the subsequent step of folding into two, the male member engages the flap of the female member. On cutting the folded web, there are produced absorbent articles with the side edge portions of the rear portion and the side edge portions of the front portion joined to each other via the respective mechanical fasteners.

The present invention achieves the above object also by the provision of an absorbent article having a liquid permeable topsheet, a liquid impermeable leak-preventive sheet, a liquid retentive absorbent member interposed between the sheets, and an exterior material extending laterally outward of both rear and front portions of the absorbent member. The absorbent article has a side edge portion of its rear portion and a side edge portion of its front portion joined via a mechanical fastener composed of a male member and a female member and therefore takes on a shape of a pair of pants. A base sheet of the male member is attached, on the reverse side of its outer edge portion, to the inner side of a side edge portion of either the rear or front portion, with the portion inward of the outer edge portion being left free as a flap. The male member is disposed on.the surface of the flap. The male member engages the female member provided on the inner side of a side edge portion of the other of the rear and front portions to join the side edge portion of the rear portion and the side edge portion of the front portion with each other.

The present invention attains the above object also by the provision of a method of producing the above-described absorbent article. The method includes the steps of continuously feeding an assembly web constituting the absorbent article in a transverse feed line, folding the web into two along a folding line parallel with the feed direction, and cutting the folded web into individual absorbent articles along predetermined cutting lines. The method further includes, before the steps of folding and cutting, the step of attaching the male member base sheet having the male member disposed thereon, on the reverse side of its outer edge portion, to the inner side of the assembly web on both sides of every predetermined cutting line corresponding to both side edge portions of either the rear or front portion of an absorbent article to be produced on cutting. In the subsequent step of folding into two, the male member engages the female member provided on both sides of every predetermined cutting line in the other of the rear and front portions. On cutting the folded web, there are produced absorbent articles with the side edge portions of the rear portion and the side edge portions of the front portion joined to each other via the respective mechanical fasteners.

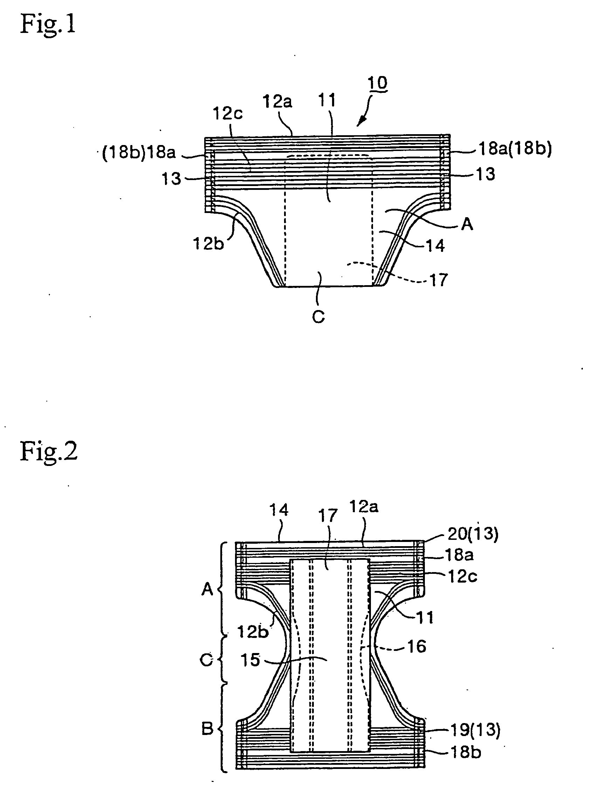

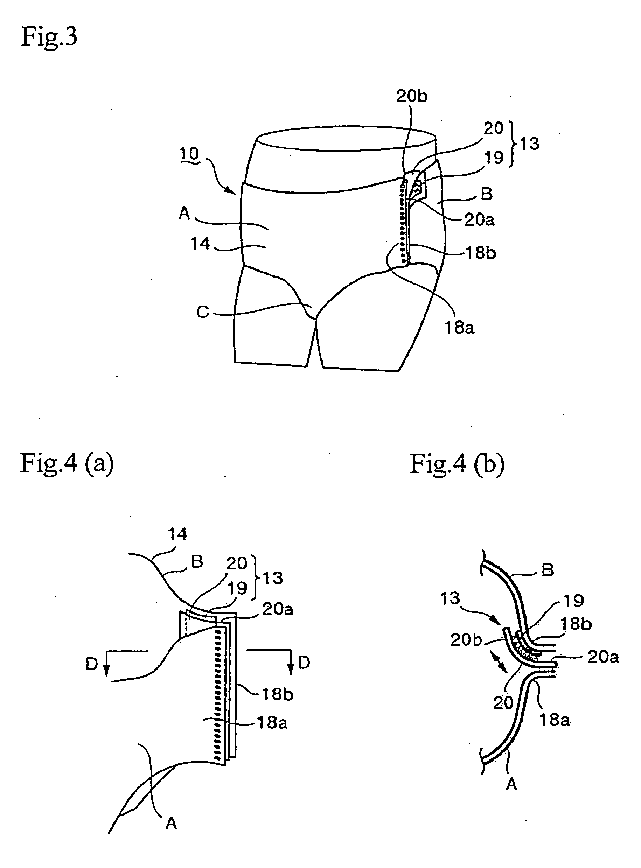

An absorbent article incorporating a first preferred embodiment of the invention is, for example, one produced as a pants-type diaper 10 as illustrated in Figs. 1 and 2. The pants-type diaper 10 is constituted by an exterior material 14 and an oblong absorbent body 17 attached to the inner side of the exterior material 14. The exterior material 14 is obtained by cutting a composite sheet having a base sheet 11 and elastic members 12a, 12b, and 12c disposed on the base sheet 11 in their stretched state. The absorbent body 17 has a liquid permeable topsheet 15, a liquid impermeable leak preventive sheet (not shown), and a liquid retentive absorbent member 16 interposed between these sheets. The pants-type diaper 10 of the present embodiment has the form of a pair of pants as a result of folding the exterior material 14 and the absorbent body 17 as a unit in two along the center of their crotch portion C to superpose the front portion A and the rear portion B on each other and joining preferably both side edge portions 18b of the rear portion B to both side edge portions 18a of the front portion A via respective mechanical fasteners 13 including a male member 19 and a female member 20 (see Figs. 3, 4(a), and 4(b)).

The pants-type diaper 10 as an absorbent article incorporating the first embodiment of the invention is constituted by, for example, the absorbent body 17 having the liquid permeable topsheet 15, the liquid impermeable leak preventive sheet (not shown), and the liquid retentive absorbent member 16 interposed between these sheets and the exterior material 14 including the rear portion B, the front portion A, and the crotch portion C. As shown in Figs. 3, 4(a), and 4(b), the pants-type diaper 10 is an absorbent article having the form of a pair of pants, the opposite side edge portions 18b of the rear portion B being joined to the facing opposite side edge portions 18a of the front portion A via the respective mechanical fasteners 13 including the male member 19 and the female member 20. The male member 19 is attached to the inner side (the skin facing side) of each side edge portion 18b of the rear portion B. The female member 20 is a sheet material having an outer edge portion 20a and an inner portion that is inward of the outer edge portion 20a. The female member 20 is attached, on the reverse side (the side facing the exterior material 14) of the outer edge portion 20a thereof, to the inner side (skin facing side) of each side edge portion 18a of the front portion A, with the inner portion being left free to form a flap 20b. Each male member 19 engages the flap 20b of each female member 20 thereby to join both side edge portions 18b of the rear portion B and both side edge portions 18a of the front portion A to each other.

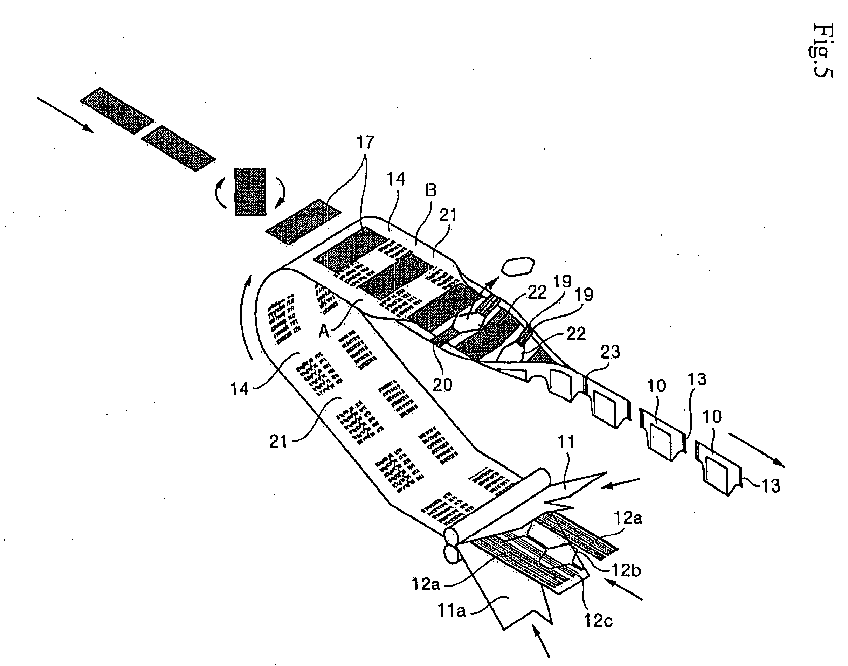

According to the first embodiment, the pants-type diaper 10 can be manufactured through almost the same steps for the manufacture of conventional pull-on diapers and additional steps hereinafter described. For example, as illustrated in Fig. 5, a web of outer nonwoven fabric is unrolled as a base sheet 11. A plurality of waist elastic members 12a, a plurality of leg elastic members 12b, and a plurality of below-waist elastic members 12c are disposed on the base sheet 11 in their stretched state at predetermined stretch ratios while continuously applying an adhesive, e.g., a hot melt adhesive to the base sheet 11. A web of inner nonwoven fabric 11a is unrolled and bonded to the base sheet 11 with the elastic members 12a, 12b, and 12c inside to form a composite sheet web 21, i.e., a continuous form of the exterior material 14 formed integrally of the base sheet 11, the elastic members 12a, 12b, and 12c, and the inner nonwoven fabric 11a.

The web 21 of the exterior material 14 is further fed on a transverse feed line in the width direction of pants-type diapers 10 to be manufactured. Absorbent bodies 17 each having a topsheet 15, a leak preventive sheet, and an absorbent member 16 are spacedly disposed on the web 21, and leg hole openings 22 are made using, for example, a leg hole cutter. The web 21 of the exterior material 14 is then folded into two along a folding line parallel with the feed direction with the absorbent bodies 17 inside the fold. The web 21 having the absorbent bodies 17 is cut into individual products, i.e., pants-type diapers 10.

As illustrated in Figs. 3, 4(a), and 4(b), the pants-type diaper 10 according to the first embodiment have their opposite side edge portions 18b of the rear portion B and their opposite side edge portions 18a of the front portion A joined together via respective mechanical fasteners 13 each including the male member 19 and the female member 20. Various male members of known mechanical fasteners can be used as the male member 19 of the mechanical fastener 13. Examples of useful male members include those having a large number of hooks, such as J hooks, mushroom hooks, T hooks, and Y hooks, standing on a side of a base. In the first embodiment, the male member 19 is a strip having a width, e.g., of about 3 to 40 mm, attached on the inner side of each side edge portion 18b of the rear portion B over almost the whole length of the side edge portion 18b except that the upper and lower ends of each side edge portion 18b are left as upper and lower margins. The attachment is done by, for example, fusion bonding (e.g., heat sealing or ultrasonic sealing) or with a hot melt adhesive to exhibit some considerable adhesion strength. The upper and lower margins of the side edge portions 18b not only function as a pinch tab in taking the pant-type diaper 10 off a wearer but also prevent a direct contact of the hook side surface, the reverse surface or the angular corners of the male member 19 with the wearer's skin. Each of the margins preferably measures 1 to 30 mm, more preferably 5 to 20 mm.

In the first embodiment, providing the male member 19 on the inner side of each side edge portion 18b of the rear portion B brings about improved disposability of the pants-type diaper 10. That is, when the diaper 10 is disposed of after use, the front portion A is compactly rolled into a ball with the rear portion B outside, and both the side edge portions 18b of the rear portion B are fastened to the outer side of the rolled pants-type diaper 10 via their male members 19. Thus, the soiled pants-type diaper 10 can be made into a compact shape without the aid of a separately provided tape for disposal. As a result, the soiled pants-type diaper 10 can be discarded as a compact waste with little emission of the order of the excrements.
The male member 19 is preferably attached at a distance not more than 30 mm from the side edge of each side edge portion 18b of the rear portion B. At this distance, the flap 20b facing the male member 19 does not have to have a wastefully excessive width, and the materials are made effective use for defining the inner circumferential side of the diaper. As a result, material waste can be avoided effectively.

The female member 20 of the mechanical fastener 13 is required from a functional aspect to show necessary engagement strength against the male member 19 while the absorbent articles are shipped as final products and to maintain the engagement strength till the end of use. In this regard, selection of the material of the female member 20 depends on the selected material of the male member 19. Whether re-engagement between the male and female members is intended is also an important factor in selecting the material of the female member 20. Another preferred important function of the female member 20 is to maintain the adhesive strength to the exterior material 14 and to have such material strength as not to break itself under the load during wear. Various female members of known mechanical fasteners can be used as the female member 20. Examples of useful female members include those having a base sheet with a large number of fibers knitted therein making loops or arches, nonwoven fabrics having a prescribed weight per unit area, and nonwoven fabrics fabricated of fibers having a prescribed fiber fineness. Understandably, the material constituting the exterior material 14 may be utilized, as it is, as the female member 20 as long as desired physical properties requirement are satisfied. To improve the atmosphere inside the diaper, breathable materials are desirable. In the first embodiment, heat fusible and extensible/contractible nonwoven fabric is preferably used as the female member 20. To use heat fusible nonwoven fabric as the female member 20 is very significant to increase productivity while securing the adhesion strength between the female member 20 and the exterior material 14. To use extensible/contractible nonwoven fabric as the female member 20 improves the fit of the pants-type diaper 10 to the wearer's body.

According to the first embodiment, the female member 20 on each side is a strip-shaped sheet having a width, e.g., of about 5 to 50 mm. The female member 20 is adhered, on the reverse side of its outer edge portion 20a, to the inner side of both the opposing side edge portions 18a of the front portion A over the almost whole length of the side edge portion 18a. The outer edge portion 20a has a width, e.g., of about 1 to 20 mm, preferably 3 to 10 mm. The adhesion is done, for example, with a hot melt adhesive or by fusion bonding. The manner of adhering the female member 20 to each side edge portion 18a can be designed as appropriate to the usage of the female member 20 and the physical properties of the materials after the adhesion. For example, the joint area is preferably minimized so as not to impair the material softness and to reduce the stiffness of the joint. If allowable, dot sealing is preferred to continuous sealing. In the case of dot sealing, it is preferred that dot seals having a circular, elliptic, rectangular, rhombic or linear shape, etc. be discretely formed. The female member 20 is preferably attached with its outer edge portion 20a close to the side edge of the side edge portion 18, whereby the material is made effective use for defining the inner circumferential side of the diaper, and the male member 19 is allowed to effectively engage the female member 20 over substantially the entire area thereof. The female member 20 is preferably provided such that the free edge of the flap 20b may extend inward over the inner edge of the male member 19 after engagement. By so designing, the surface of the male member 19 is completely covered with the female member 20 without sticking out of the free edge of the female member 20 so that the hooks of the male member 19 are effectively prevented from directly contacting the skin to cause discomfort.

In the first embodiment, the adhesive strength between the outer edge portion 20a of the female member 20 and the inner side of the side edge portion 18a of the front portion A is preferably 3 N/30 mm or higher, more preferably 5 N/30 mm or higher, even more preferably 8 N/30 mm or higher. Unlike the joint of the mechanical fastener 13 by the engagement between the male member 19 and the female member 20, the joint between the outer edge portion 20a of the female member 20 and the inner side of the side edge portion 18a will receive a pulling force in the direction of T while the pants-type diaper 10 is worn. When the adhesive strength of this joint is 3 N/30 mm or higher, the joint is able to effectively withstand the pulling force imposed in the direction of T.

The waist elastic members 12a, the leg elastic members 12b, and the below-waist elastic members 12c are disposed on both the side edge portions 18b of the rear portion B and the side edge portions 18a of the front portion A. In the first embodiment, at least part of these elastic members 12a, 12b, and 12c are made to have no substantial elastic contractibility in the sites where the male member 19 and the female member 20 are to be attached by, for example, cutting or heat-sealing the elastic members 12a, 12b and 12c.

Means useful to make the waist elastic members 12a, the leg elastic members 12b, and the below-waist elastic members 12c lose their contracting function are exemplified by one having practically the same structure as an elastic member segmentalizing section used in the production of a composite elastic material disclosed, e.g., in JP 2002-253605A. The elastic member segmentalizing section disclosed includes a cutting roll and an anvil roll with a smooth surface facing the cutting roll. The cutting roll has a pressing region on its peripheral surface. The pressing region has arranged therein a large number of projections for cutting the elastic members. The web 21 of the exterior material 14, i.e., the base sheet 11 having the elastic members 12a, 12b, and 12c disposed thereon in their stretched state is introduced into the nip of the rolls. While the web 21 is passing between the rolls, the pressing force from the pressing region is applied to the sites of the web 21 where the male members 19 and the female members 20 are to be attached (hereinafter referred to as the preset sites). As a result, the elastic members 12a, 12b, and 12c are each cut into segments to lose continuity in the preset sites with substantially minimized damage to the web 21. The above-described means will hereinafter be called a precutting means. A precutting means using a precutting cutter having cutting blades in place of the projections may also be used.

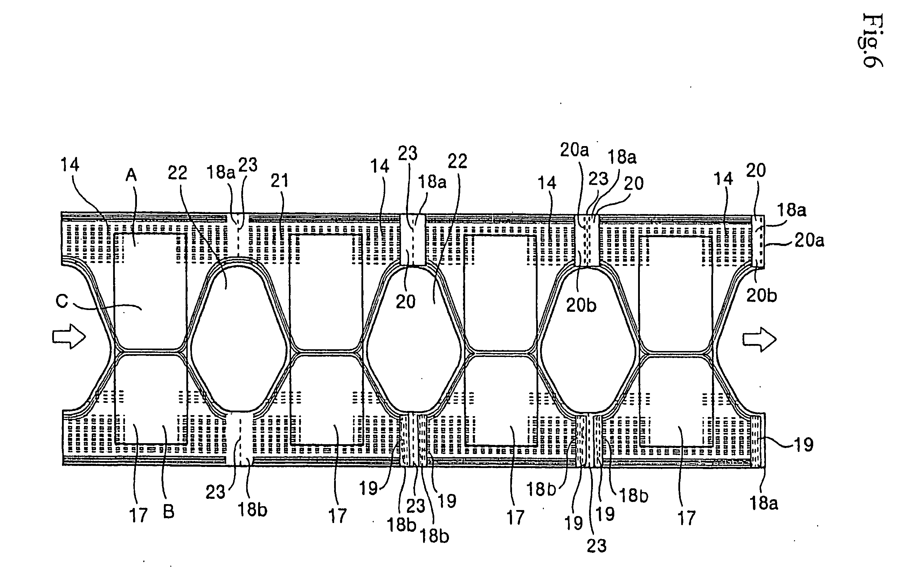

In the manufacture of the pants-type diaper 10, the waist elastic members 12a, the leg elastic members 12b, and the below-waist elastic members 12c are all released from tension upon separating the web 21 along the predetermined cutting lines 23 (see Fig. 6) into individual products. By previously making these elastic members functionless in the preset sites for the attachment of the male members 19 and the female members 20, the preset sites are effectively prevented from contracting and curling that will ruin the product appearance and ease of use.

It is preferred that the regions adjoining the sites where the waist elastic members 12a, the leg elastic members 12b, and the below-waist elastic members 12c are segmentalized to lose their function be additionally provided with a fixing means, such as a hot melt adhesive, for preventing the elastic members 12a, 12b, and 12c from being retracted. To apply a hot melt adhesive, etc. directly to the elastic members 12a, 12b, and 12c is effective to improve the adhesive strength.

In the pants-type diaper 10 incorporating the first embodiment, the inner side of the opposite side edge portions 18b of the rear portion B are face-to-face joined with the inner side of the opposite side edge portions 18a of the front portion A by means of the respective mechanical fasteners 13 each composed of the male member 19 and the female member 20. According to this configuration, a pulling force generated while the diaper 10 is worn is made to be exerted to the joints in the shearing direction without involving wasteful use of the female member 20. As a result, a sufficient joint strength can be secured easily and efficiently. More specifically, in the first embodiment, the male member 19 is attached to the inner side of the side edge portion 18b of the rear portion B, and the female member 20 is attached, on the reverse side of its outer edge portion 20a, to the inner side of the side edge portion 18a of the front portion A as illustrated in Figs. 3, 4(a), and 4(b). The part inward of the outer edge portion 20a of the female member 20 is left free as a flap 20b. The male member 19 engages the flap 20b of the female member 20. When the pants-type diaper 10 is put on a wearer, the flap 20b on which the male member 19 is caught is laid flat on the side of the rear portion B to extend along the circumferential direction of the wearer's body. As a result, the pulling force generated during wear is made to be imposed in the shearing direction efficiently and effectively with no need to use a C-folded female member having double the effective size.

The pants-type diaper 10 having the above-described configuration of the first embodiment can easily be produced by a simple method as illustrated in Figs. 5 and 6. As previously stated, the method includes the steps of continuously feeding, in a transverse feed line, an assembly web 21 of the exterior material 14 having spacedly disposed thereon absorbent bodies 17 each having a topsheet 15 (see Fig. 2), a leak preventive sheet, and an absorbent member 16 (see Fig. 2), folding the web 21 into two along a folding line parallel with the feed direction with the absorbent bodies 17 inside, and cutting the web 21 into individual absorbent articles 10 similarly to conventional production steps.

The production method of the first embodiment further includes, before the steps of folding and cutting the web 21 into individual pants-type diapers 10, the step of attaching a male member 19 to the inner side of the assembly web 21 in predetermined sites on both sides of every predetermined cutting line 23 corresponding to both side edge portions 18b of the rear portion B of an absorbent article 10 to be produced on cutting via, for example, a hot melt adhesive, and attaching the female member 20 on the outer edge portion 20a thereof to the inner side of the assembly web 21 in predetermined sites on both sides of every predetermined cutting line 23 corresponding to both side edge portions 18a of the front portion A of an absorbent article 10 to be produced on cutting via, for example, dot seals, while leaving the flap 20b thereof extending inward in a free state. It is possible that the female member 20 and the male member 19 are fed to the rear portion B or the front portion A after they are united.

On folding the web 21 into two, the male member 19 engages the flap 20b of the female member 20, and the web 21 is then cut along every predetermined cutting line 23 into individual products. In that way, pants-type diaper 10 as an absorbent article incorporating the first embodiment, in which the opposite side edge portions 18b of the rear portion B and the opposite side edge portions 18a of the front portion A are joined together via the respective mechanical fasteners 13, can easily be produced. The production can be achieved through practically the same simple steps as in the conventional production method using a transverse feed line without involving such a complicated step of folding the female member in two. When the web 21 is folded in two, and the male member 19 and the flap of the female member 20 are engaged with each other, it is preferred to provide a complementary pressing means to enhance the engagement. In the case where the complementary pressing means is a pair of rolls for pressure application, pressure may be applied only to the engagement sites while making a difference in peripheral speed between the rolls to impose a shear force thereby to improve the engagement strength.

In the production method of the first embodiment, the following manner of mechanical fastener attachment is preferred. Two pieces of the male member 19 are separately attached on the respective sides of every predetermined cutting line 23 in the rear portion B of the web 21. On the other hand, one piece of the female member 20 is attached to straddle every predetermined cutting line 23 in the front portion A of the web 21. On cutting the web 21 along every predetermined cutting line 23, cutting is carried out such that a cut piece of the female member 20 is provided on both side edge portions 18a of the front portion A of the pants-type diapers 10. By separately attaching two pieces of the male member 19, the two side edges of the rear portion B can get rid of the male member 19 (existence of the male member on the side edges would often cause a wearer discomfort), and the male member whose unit price is high can be saved, which leads to reduction of material cost. Attaching one-piece female member 20, on the other hand, facilitates handling of the female member 20 that is required to provide a free edge. Furthermore, attaching one-piece female member 20 is simpler than attaching individual female members 20 on both sides of the predetermined cutting line 23. The joint area between the female member 20 and the exterior material 14 may overlap the predetermined cutting line 23 but is preferably on each side of the cutting line 23, avoiding the cutting line 23 so that the hardness of the edges along the cutting line 23 can be reduced to provide a wearer-friendly texture.

The production method of the first embodiment may further include the step of temporarily fixing the flap 20b of the female members 20 that is attached to both sides of every predetermined cutting line 23 in the front portion A of the web 21. It is essential for the free portion of the female member 20 not to be joined to the web 21. Accordingly, the expression "temporarily fixing" as used herein means that the flap of the female member 20 is stuck to the web 21 to an easily detachable degree in use so as not to flip up during the production to cause any inconvenience. Known means such as a hot melt adhesive, heat fusion sealing, and press bonding can be used for temporary fixing. The temporary fixing to the web 21 is preferably to such a degree that is not accompanied by adherend failure. The temporary fixing effectively prevents the flap 20 from flipping up during the production of the pants-type diaper 10.

Preferably, the production method of the first embodiment further includes the step of making the elastic members 12a, 12b, and 12c functionless in the preset sites by precutting them as described above prior to the step of attaching the male member 19 and the female member 20 on both sides of every predetermined cutting line 23 of the web 21. Making the elastic members functionless in the sites of the exterior material 14 where the male member 19 is to be disposed is particularly desirable taking engagement with the flap of the female member 20 into consideration.

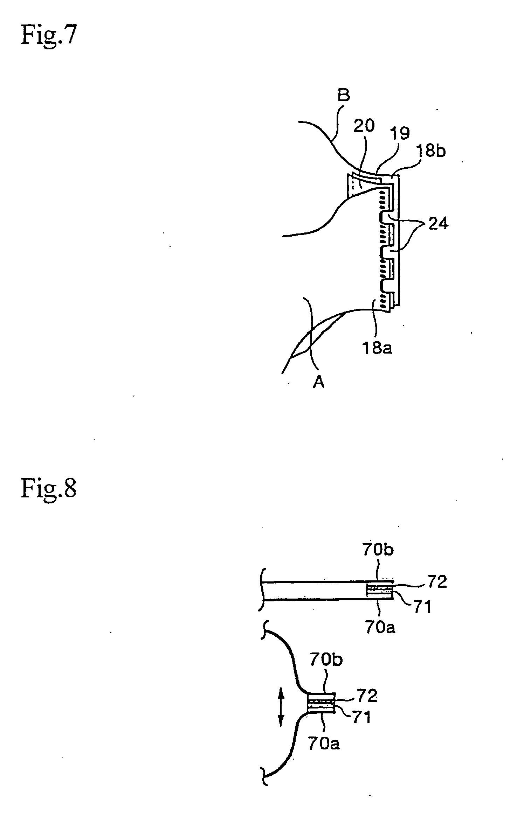

The present invention is not limited to the first embodiment, and various changes and modifications can be made therein. For instance, the present invention is applicable to not only pants-type diapers but also other absorbent articles having the form of a pair of pants. While the first embodiment has been described with reference to a pants-type diaper constituted by an absorbent body and an exterior material, the present invention is applicable to a pants-type diaper having no absorbent body. The absorbent article of the invention is not limited to the one produced by the method of the first embodiment and may be produced by any other method, including not only the transverse feed system but a longitudinal feed system. The joint by the mechanical fastener may be constituted between a male member attached to the side edge portions of the front portion and a female member attached to the side edge portions of the rear portion. In taking a soiled diaper off a wearer, the side engagement can easily be released by applying a force to the mechanical fastener in a peeling direction. To help release the engagement, it is preferred as previously mentioned that the male member is laid to leave margins so that the upper margin may serve as a pinch tab. As another means for helping release the engagement of the mechanical fastener, a pinch tab 24 may be formed by cutting U-shaped notches along the side edge portion 18a of the front portion A as illustrated in Fig. 7. The pinch tab 24 makes the side edge portion 18b of the rear portion B easier to pinch. In case re-engagement is needed, such a design facilitates re-engagement. The absorbent article of the present invention can have both the opposite side edge portions of the rear portion joined to both the opposite side edge portions of the front portion by respective mechanical fasteners each composed of a male member and a female member having the above-described structure or can have only one of the side edge portions of the rear portion joined to the facing side edge portion of the front portion by the above-described mechanical fastener.

### Industrial Applicability

The present invention provides an absorbent article and a method of producing the absorbent article, in which the rear side edge portion and front side edge portion are joined together on their inner side by mechanical fastener engagement between a male member and a female member, and yet a pulling force during wear is exerted in the shearing direction. The absorbent article can easily be produced through simple steps without wasteful use of the female member.

## Claims

1. An absorbent article comprising a liquid permeable topsheet (15), a liquid impermeable leak-preventive backsheet, a liquid retentive absorbent member (16) intermediate between the topsheet and the backsheet, and an exterior material (14) extending laterally outward of both rear and front portions of the absorbent member (16), the absorbent article having a side edge portion (18b) of the rear portion (B) thereof and a side edge portion (18a) of the front portion (A) thereof joined to each other via a mechanical fastener (13) comprising a male member (19) and a female member (20) to take on the shape of a pair of pants,
the male member (19) being attached to the inner side of the side edge portion (18a, 18b) of one of the rear portion (B) and the front portion (A),
the female member (20) being a sheet member attached to the inner side of the side edge portion (18a, 18b) of the other of the rear portion (B) and the front portion (A) on the reverse side of its outer edge portion (20a), with the portion of the female member (A) inward of the outer edge portion (20a) left free as a flap (20b), and
the male member (19) engaging the flap (20b) of the female member (20) to join the side edge portion (18b) of the rear portion (B) and the side edge portion (18a) of the front portion (A) with each other,
the absorbent article further comprising waist elastic members (12a) and below-waist elastic members (12c), the waist elastic members (12a) and below-waist elastic members (12c) having no contracting function in the site where the male member (19) is attached and/or the site where the female member (20) is attached.

2. The absorbent article according to claim 1, wherein the male member (19) is attached to the side edge portion (18b) of the rear portion (B), and the female member (20) is attached to the side edge portion (18a) of the front portion (A).

3. A method of producing the absorbent article of claim 1, comprising the steps of continuously feeding an assembly web constituting the absorbent article in a transverse feed line, folding the web into two along a folding line parallel with the feed direction, and cutting the folded web along predetermined cutting lines (23) into individual absorbent articles (10),
the method further comprising the steps of making the elastic members (12a, 12c) functionless in said sites by pre-cutting them, and attaching one of the male (19) and female members (20) to both sides of each predetermined cutting line (23) in one of the rear (B) and front portions (A) of the assembly web and attaching the other of the male and female members (19, 20) to both sides of each predetermined cutting line (23) in the other of the rear and front portions (A, B) of the assembly web before the steps of folding and cutting, the sites where the male and female members (19, 20) are attached corresponding to the inner side of both side edge portions (18b, 18a) of one of the rear and front portions (B, A) and the other, respectively, of each absorbent article to be produced on cutting,
the step of folding the web into two causing each male member (19) to engage the flap (20b) of each facing female member (20), and
the step of cutting the folded web providing a plurality of absorbent articles having the side edge portions (18b) of the rear portion (B) and the side edge portions (18a) of the front portion (A) joined to each other via the respective mechanical fasteners (13).

4. The method of producing the absorbent article of claim 3, wherein the female member (20) is attached to straddle every predetermined cutting line (23), and, before or simultaneously with the web being cut along every predetermined cutting line (23), the female member (20) is provided on both side edge portions of the rear or front portion (B, A) of the individual absorbent articles.

5. The method of producing the absorbent article of claim 3 or 4, further comprising the step of temporarily fixing the flap (20b) of the female member (20) attached to each side of the predetermined cutting line (23).

## Patentansprüche

1. Saugfähiger Artikel mit einer flüssigkeitsdurchlässigen Decklage (15), einer flüssigkeitsundurchlässigen auslaufsicheren Trägerlage, einem flüssigkeitshaltenden saugfähigen Teil (16) zwischen der Decklage und der Trägerlage und einem Außenmaterial (14), das sich sowohl von einem hinteren als auch von einem vorderen Abschnitt des saugfähigen Teils (16) seitlich nach außen erstreckt, wobei der saugfähige Artikel einen Seitenkantenabschnitt (18b) seines hinteren Abschnitts (B) und einen Seitenkantenabschnitt (18a) seines vorderen Abschnitts (A) hat, die über eine mechanische Befestigung (13) mit einem männlichen Teil (19) und einem weiblichen Teil (20) so miteinander verbunden werden, dass sie die Form eines Höschens annehmen,
wobei das männliche Teil (19) an der Innenseite des Seitenkantenabschnitts (18a, 18b) einer Komponente aus dem hinteren Abschnitt (B) und dem vorderen Abschnitt (A) angebracht ist,
wobei das weibliche Teil (20) ein Lagenteil ist, das an der Innenseite des Seitenkantenabschnitts (18a, 18b) der anderen Komponente aus dem hinteren Abschnitt (B) und dem vorderen Abschnitt (A) auf der Rückseite seines Außenkantenabschnitts (20a) angebracht ist, wobei der Abschnitt des weiblichen Teils (20) einwärts vom Außenkantenabschnitt (20a) als Klappe (20b) frei bleibt, und wobei das männliche Teil (19) einen Eingriff mit der Klappe (20b) des weiblichen Teils (20) herstellt, um den Seitenkantenabschnitt (18b) des hinteren Abschnitts (B) und den Seitenkantenabschnitt (18a) des vorderen Abschnitts (A) miteinander zu verbinden,
wobei der saugfähige Artikel ferner an der Taille liegende elastische Teile (12a) und unter der Taille liegende elastische Teile (12c) aufweist, wobei die an der Taille liegenden elastischen Teile (12a) und unter der Taille liegenden elastischen Teile (12c) keine zusammenziehende Funktion an der Stelle, an der das männliche Teil (19) angebracht ist, und/oder an der Stelle haben, an der das weibliche Teil (20) angebracht ist.

2. Saugfähiger Artikel nach Anspruch 1, wobei das männliche Teil (19) am Seitenkantenabschnitt (18b) des hinteren Abschnitts (B) angebracht ist und das weibliche Teil (20) am Seitenkantenabschnitt (18a) des vorderen Abschnitts (A) angebracht ist.

3. Verfahren zur Herstellung des saugfähigen Artikels nach Anspruch 1 mit den Schritten des kontinuierlichen Zuführens einer den saugfähigen Artikel bildenden Zusammenstellungsbahn in einer Querzufuhrlinie, einmaligen Faltens der Bahn entlang einer Faltlinie parallel zur Zufuhrrichtung und Schneidens der gefalteten Bahn entlang vorbestimmter Schnittlinien (23) in einzelne saugfähige Artikel (10),
wobei das Verfahren ferner die Schritte aufweist: Funktionslosmachen der elastischen Teile (12a, 12c) an den Stellen durch ihr vorab erfolgendes Schneiden sowie Anbringen einer Komponente aus dem männlichen (19) und weiblichen Teil (20) an beiden Seiten jeder vorbestimmten Schnittlinie in einer Komponente aus dem hinteren (B) und vorderen Abschnitt (A) der Zusammenstellungsbahn und Anbringen der anderen Komponente aus dem männlichen und weiblichen Teil (19, 20) an beiden Seiten jeder vorbestimmten Schnittlinie (23) in der anderen Komponente aus dem hinteren und vorderen Abschnitt (A, B) der Zusammenstellungsbahn vor den Schritten des Faltens und Schneidens, wobei die Stellen, an denen das männliche und weibliche Teil (19, 20) angebracht werden, der Innenseite beider Seitenkantenabschnitte (18b, 18a) einer Komponente aus dem hinteren und vorderen Abschnitt (B, A) bzw. der anderen Komponente jedes nach Schneiden herzustellenden saugfähigen Artikels entsprechen,
wobei der Schritt des einmaligen Faltens der Bahn bewirkt, dass jedes männliche Teil (19) einen Eingriff mit der Klappe (20b) jedes gegenüberliegenden weiblichen Teils (20) herstellt, und
wobei der Schritt des Schneidens der gefalteten Bahn mehrere saugfähige Artikel bereitstellt, bei denen die Seitenkantenabschnitte (18b) des hinteren Abschnitts (B) und die Seitenkantenabschnitte (18a) des vorderen Abschnitts (A) über die jeweiligen mechanischen Befestigungen (13) miteinander verbunden sind.

4. Verfahren zur Herstellung des saugfähigen Artikels nach Anspruch 3, wobei das weibliche Teil (20) so angebracht wird, dass es jede vorbestimmte Schnittlinie (23) überspannt, und vor oder gleichzeitig mit dem Schneiden der Bahn entlang jeder vorbestimmten Schnittlinie (23) das weibliche Teil (20) auf beiden Seitenkantenabschnitten des hinteren oder vorderen Abschnitts (B, A) der einzelnen saugfähigen Artikel bereitgestellt wird.

5. Verfahren zur Herstellung des saugfähigen Artikels nach Anspruch 3 oder 4, ferner mit dem Schritt des zeitweiligen Fixierens der Klappe (20b) des weiblichen Teils (20), das an jeder Seite der vorbestimmten Schnittlinie (23) angebracht ist.

## Revendications

1. Article absorbant comprenant une feuille supérieure (15) perméable aux liquides, une feuille arrière anti-fuite imperméable aux liquides, un élément absorbant (16) à retenue de liquides entre la feuille supérieure et la feuille arrière, et un matériau extérieur (14) s'étendant latéralement vers l'extérieur des deux parties avant et arrière de l'élément absorbant (16), une partie bord latéral (18b) de la partie arrière (B) de l'article absorbant et une partie bord latéral (18a) de la partie avant (A) de l'article absorbant étant reliées l'une à l'autre par le biais d'un élément de fixation mécanique (13) comprenant un élément mâle (19) et un élément femelle (20) pour prendre la forme d'un slip,
l'élément mâle (19) étant attaché au côté intérieur de la partie bord latéral (18a, 18b) d'une partie parmi la partie arrière (B) et la partie avant (A),
l'élément femelle (20) étant un élément de feuille attaché au côté intérieur de la partie bord latéral (18a, 18b) de l'autre partie parmi la partie arrière (B) et la partie avant (A) sur le côté opposé de sa partie bord extérieur (20a), la partie de l'élément femelle (20) située à l'intérieur de la partie bord extérieur (20a) restant libre et servant de rabat (20b), et
l'élément mâle (19) coopérant avec le rabat (20b) de l'élément femme (20) pour relier la (B) et la partie bord latéral (18a) de la partie avant (A) l'une à l'autre,
l'article absorbant comprenant en outre des éléments élastiques pour taille (12a) et des éléments élastiques sous la taille (12c), les éléments élastiques pour taille (12a) et les éléments élastiques sous la taille (12c) n'ayant aucune fonction de contraction à l'endroit où l'élément mâle (19) est attaché et/ou à l'endroit où l'élément femelle (20) est attaché.

2. Article absorbant selon la revendication 1, dans lequel l'élément mâle (19) est attaché à la partie bord latéral (18b) de la partie arrière (B), et l'élément femelle (20) est attaché à la partie de bord latéral (18a) de la partie avant (A).

3. Procédé de fabrication de l'article absorbant selon la revendication 1, comprenant les étapes consistant à acheminer en continu une bande d'ensemble constituant l'article absorbant dans une ligne d'acheminement transversal, à plier la bande en deux le long d'une ligne de pliage parallèle à la direction d'acheminement, et à découper la bande pliée le long de lignes de découpe prédéfinies (23) en articles absorbants individuels (10),
le procédé comprenant en outre les étapes consistant à rendre les éléments élastiques (12a, 12c) inactifs auxdits endroits en les prédécoupant, et à attacher un élément parmi l'élément mâle (19) et l'élément femelle (20) aux deux côtés de chaque ligne de découpe prédéfinie (23) dans une partie parmi la partie arrière (B) et la partie avant (A) de la bande d'ensemble et à attacher l'autre élément parmi l'élément mâle (19) et l'élément femelle (20) aux deux côtés de chaque ligne de découpe prédéfinie (23) dans l'autre partie parmi la partie arrière (B) et la partie avant (A) de la bande d'ensemble avant les étapes de pliage et de découpe, les endroits où les éléments mâle et femelle (19, 20) sont attachés correspondant au côté intérieur des deux parties de bord latéral (18b, 18a) de l'une des parties avant et arrière (B, A) et de l'autre, respectivement, de chaque article absorbant à produire lors de la découpe,
l'étape de pliage en deux de la bande amenant chaque élément mâle (19) à coopérer avec le rabat (20b) de chaque élément femelle (20) opposé, et
l'étape de découpe de la bande pliée créant une pluralité d'articles absorbants dont les parties bord latéral (18b) de la partie arrière (B) et les parties bord latéral (18a) de la partie avant (A) sont reliées les unes aux autres par le biais des éléments de fixation mécaniques (13) respectifs.

4. Procédé de production de l'article absorbant selon la revendication 3, dans lequel l'élément femelle (20) est attaché pour chevaucher chaque ligne de découpe prédéfinie (23), et, avant la découpe de la bande le long de chaque ligne de découpe prédéfinie (23) ou en même temps que celle-ci, l'élément femelle (20) se trouve sur les deux parties bord latéral de la partie arrière ou avant (B, A) des articles absorbants individuels.

5. Procédé de production de l'article absorbant selon la revendication 3 ou 4, comprenant en outre l'étape consistant à fixer provisoirement le rabat (20b) de l'élément femelle (20) attaché à chaque côté de la ligne de découpe prédéfinie (23).
